# EUROPEAN PATENT SPECIFICATION

(11) **EP 2 486 882 B1**
(45) Date of publication and mention of the grant of the patent: **20.04.2016**
(21) Application number: 12154733.5
(22) Date of filing: 09.02.2012
(51) Int. Cl.: A61B 17/34, A61B 17/02

(54) **Thoracic access port**
Brustkorbzugangsöffnung
Port d'accès thoracique

(30) Priority: 14.02.2011 US 201161442399 P; 18.01.2012 US 201213353088
(43) Date of publication of application: 15.08.2012
(73) Proprietor: Covidien LP, Mansfield, MA 02048 (US)
(72) Inventor: O'Prey, Cormac, Bishops Stortford, Hertfordshire CM23 4HH (GB); Cauwood, Peter David, Cambridge CB25 9HP (GB); Thompson, Keith Paul, Ely, Cambridgeshire CB6 1FX (GB)
(74) Representative: Soames, Candida Jane

(56) References cited:
- EP-A1- 1 707 126
- EP-A1- 2 462 883
- EP-A2- 2 422 725
- WO-A1-02/091953
- WO-A2-2010/042913
- US-A1- 2004 199 200
- US-A1- 2011 021 879

## Description

### BACKGROUND

### Technical Field

The present disclosure relates generally to devices and techniques for performing surgical procedures. More particularly, the present disclosure relates to reinforced access devices for minimally invasive surgery.

### Background of the Related Art

In an effort to reduce trauma and recovery time, many surgical procedures are performed through small openings in the skin, such as an incision or a natural body orifice. For example, these procedures include laparoscopic procedures, which are generally performed within the confines of a patient's abdomen, and thoracic procedures, which are generally performed within a patient's chest cavity.

Specific surgical instruments have been developed for use during such minimally invasive surgical procedures. These surgical instruments typically include an elongated shaft with operative structure positioned at a distal end thereof, such as graspers, clip appliers, specimen retrieval bags, etc.

During minimally invasive procedures, the clinician creates an opening in the patient's body wall, oftentimes by using an obturator or trocar, and thereafter positions an access assembly within the opening. The access assembly includes a passageway extending therethrough to receive one or more of the above-mentioned surgical instruments for positioning within the internal work site, e.g. the body cavity.

During minimally invasive thoracic procedures, an access assembly is generally inserted into a space located between the patient's adjacent ribs that is known as the intercostal space, and then surgical instruments can be inserted into the internal work site through the passageway in the access assembly.

In the interests of facilitating visualization, the introduction of certain surgical instruments, and/or the removal of tissue specimens during minimally invasive thoracic procedures, it may be desirable to spread the adjacent ribs defining the intercostal space and/or retract soft tissue. Additionally, during these procedures, firm, reliable placement of the access assembly is desirable to allow the access assembly to withstand forces that are applied during manipulation of the instrument(s) inserted therethrough. However, reducing patient trauma during the procedure, discomfort during recovery, and the overall recovery time remain issues of importance. Thus, there exists a need for thoracic access ports which minimize post operative patient pain while enabling atraumatic retraction of tissue and which do not restrict access to the body cavity, as well as facilitates removal of tissue specimens from the body cavity. US 2011/021879 discloses a surgical access device comprising:
a proximal end, a distal end, a longitudinal axis extending from the proximal end to the distal end, and an access channel extending therethrough;
a wound retractor comprising an outer ring, an inner ring, and a tubular sheath extending between the outer ring and the inner ring; and
an internal retractor coupled to the wound retractor in an operative state of the surgical access device, wherein the operative state, at least a portion of the internal retractor extends distally of the inner ring.

### SUMMARY

There is disclosed a surgical access assembly for positioning within an opening in tissue. The surgical access assembly generally includes a flexible body portion positionable within a patient and having an opening and at least one reinforcing member. The body portion is deformable to a first position for insertion. A flexible member is coupled to the flexible body portion and extends proximally therefrom.

The at least one reinforcing member includes first and second reinforcing panels separated by a web portion. In some embodiments, the reinforcing panels can be within the flexible body portion, e.g. overmolded.

In some embodiments, the at least one reinforcing member can include opposed pairs of reinforcing panels at opposite ends of the flexible body portion. The opposed pairs of reinforcing panels can have a curved configuration.

The at least one reinforcing member can include opposed pairs of side reinforcing panels.

The reinforcing panels taper from a greater thickness adjacent the periphery of the flexible body portion to a thinner thickness adjacent the opening.

The flexible body portion can include an inner ring defining the opening which can be centrally positioned.

In some embodiments, the at least one reinforcing member is an end rib. In some embodiments, the end ribs can be curved. In some embodiments, the at least one reinforcing member is an opposed pair of end ribs.

The at least one reinforcing member in some embodiments is a side rib. The side rib can be curved. The side rib in some embodiments is connected to the inner ring. In some embodiments, the side rib has a bat wing shape. The bat wing shaped rib can be skeletonized; in other embodiments, it can be monolithic.

The surgical access assembly can include an outer ring attached to a proximal portion of the flexible member.

There is further disclosed in another aspect a surgical access assembly for positioning within an opening in tissue. The surgical access assembly includes a collapsible flexible body portion having a tensioning mechanism. The tensioning mechanism includes a plurality of ferrules provided about the periphery of the body portion and a tensioning string freely passing through the ferrules. The surgical access assembly further includes a flexible member coupled to the flexible body portion.

The tensioning mechanism can additionally include an anchoring ferrule having a bore for receipt of a free end of the tensioning string and a through bore for passage of the tensioning string.

There is still further disclosed in another aspect a surgical access port having an offset spacer ring and a flexible member. The offset spacer ring includes first and second U-shaped members. The first U-shaped member has a first portion and a first arm extending from the first portion, the first arm terminating in an opening. The second U-shaped member has a second portion and a second arm extending from the second portion, the second arm terminating in an insertion tip. The insertion tip is insertable into the opening in the first arm of the first portion. The offset spacer is movable to separate the U-shaped members to expand the ring and liftable away from the skin to tension the membrane.

### BRIEF DESCRIPTION OF THE DRAWINGS

Various embodiments of the subject access port are described herein with reference to the drawings wherein:
FIG. 1 is a front view illustrating a patient's skeletal structure with one embodiment of a surgical access port in accordance with the present disclosure positioned within the intercostal space defined between adjacent ribs;
FIG. 2 is a perspective view of a lower ring or body of the surgical access port of FIG. 1, incorporating reinforcing panels;
FIG. 3 is a top plan view of the body of FIG. 2;
FIG. 4 is a cross-sectional view taken along line A-A of FIG. 3;
FIG. 5 is a cross-sectional view taken along line B-B of FIG. 3;
FIG. 6 is a perspective view of the surgical access port of FIG. 1;
FIG. 7 is a cross-sectional view of the surgical access port of FIG. 6;
FIG. 8 is a perspective view of the surgical access port of FIG. 6 positioned above an incision in a patient's body;
FIG. 9 is a perspective view illustrating the body of the surgical access port of FIG. 6 being folded and inserted into the incision in the patient's body, the other portions of the port have been removed for clarity;
FIG. 10 is a perspective view, taken from below, of the body of the surgical access port being inserted through the incision in the patient's body;
FIG. 11 is a perspective view, taken from below, of the body of the surgical access port seated against the inner surface of the patient's body and against the adjacent ribs;
FIG. 12 is a perspective view, taken from above, illustrating an outer ring and membrane of the surgical access port of FIG. 6 and further illustrating in phantom the body of the access port positioned in the incision through the patient's body, the surgical access port shown in an untensioned (unexpanded) condition;
FIG. 13 is cross-sectional view illustrating the surgical access port of FIG. 6 positioned through the incision in the patient's body and in the untensioned condition corresponding to the position of FIG. 12;
FIG. 14 is a perspective view of the surgical access port of FIG. 6 with the outer ring being tensioned by the user to urge the body of the access port against the inner surface of the patient's body and to tension the membrane;
FIG. 15 is a cross-sectional view of the surgical access port of FIG. 6 illustrating the body of the access port urged against the inner surface of the patient's body and the membrane in the tensioned condition;
FIG. 16 is a perspective view of an alternate body of a surgical access port, incorporating reinforcing ribs;
FIG. 17A is a perspective view of a further alternate body of a surgical access port, incorporating an alternative reinforcing rib layout;
FIG. 17B is a perspective view of a still further alternate body of a surgical access port, incorporating a further alternative reinforcing rib layout;
FIG. 17C is a perspective view of a yet further alternate body of a surgical access port, incorporating a further alternate reinforcing rib layout;
FIG. 18A is a perspective view of another alternate body of a surgical access port, incorporating alternate reinforcing rib layouts;
FIG. 18B is a perspective view of a still further alternate body of a surgical access port, incorporating alternate reinforcing rib layouts;
FIG. 18C is a perspective view of yet another alternate body of a surgical access port, incorporating another alternate reinforcing rib layouts;
FIG. 19A is a perspective view of an alternative body of a surgical access port incorporating alternative reinforcing rib structure;
FIG. 19B is a perspective view of a further alternative body of a surgical access port incorporating further alternative reinforcing rib structure;
FIG. 20 is a perspective view of the access port body of FIG. 16;
FIG. 21 is a perspective view of the reinforcing ribs of the access port body of FIG. 20 removed from the remainder of the body;
FIG. 22A is a top view of an alternative body of a surgical access port, shown in a collapsed (untensioned) condition;
FIG. 22B is a perspective view of the access port body of FIG. 22A in the expanded or tensioned condition;
FIG. 22C is a side view, partially shown in section, of the tensioning string and ferrules of the body of FIG. 22A;
FIG. 23A is a perspective view, taken from above, of an alternative body of a surgical access port;
FIG. 23B is a perspective view, taken from below, of the access port body of FIG. 23A;
FIG. 23C is a perspective view, taken from below, of the access port body of FIG. 23A positioned in a body of a patient;
FIG. 23D is a perspective view, taken from above, of the body of the surgical access port of FIG. 23A being folded and inserted through an incision in the body of the patient;
FIG. 23E is a perspective view, taken from below, of the body of FIG. 23A inserted through the incision;
FIG. 23F is a perspective view, taken from below, of the body of FIG. 23A being inserted in the underside of tissue and seated against the inner surface of the patient's body;
FIG. 24A is a top view of an alternative peripheral configuration of a body of a surgical access port;
FIG. 24B is a top view of a further alternative peripheral configuration of a body of a surgical access port;
FIG. 24C is a top view of a still further alternative peripheral configuration of a body of a surgical access port;
FIG. 25A is a top view of another alternate peripheral configuration of a body of a surgical access port;
FIG. 25B is a top view of a further alternate peripheral configuration of a body of a surgical access port;
FIG. 25C is a top view of a still further alternate peripheral configuration of a body of a surgical access port;
FIG. 26A is a top view of another alternate peripheral configuration of a body of a surgical access port;
FIG. 26B is a top view of a further additional alternate peripheral configuration of a body of a surgical access port;
FIG. 27 is a perspective view of a surgical access port with an offset spacer ring in a lowered condition;
FIG. 28 is a side view, shown in section, of the offset spacer ring of the surgical access port in the lowered condition;
FIG. 29 is a perspective view of the offset spacer ring and a membrane of the surgical access port of FIG. 27 being tensioned to a raised condition; and
FIG. 30 is a side view, shown in section, of the offset spacer ring and membrane of FIG. 29 in the raised condition.

### DETAILED DESCRIPTION

Various embodiments of the presently disclosed access assembly, or access port, and methods of using the same, will now be described in detail with reference to the drawings wherein like references numerals identify similar or identical elements. In the drawings, and in the following description, the term "proximal" should be understood as referring to the end of the access port, or component thereof, that is closer to the clinician during proper use, while the term "distal" should be understood as referring to the end that is further from the clinician, as is traditional and conventional in the art. Additionally, use of the term "tissue" hereinbelow should be understood to encompass both the patient's ribs, and any surrounding tissues. It should be also be understood that the term "minimally invasive procedure" is intended to include surgical procedures through small openings/incisions performed within a confined space such as the thoracic cavity or abdominal cavity.

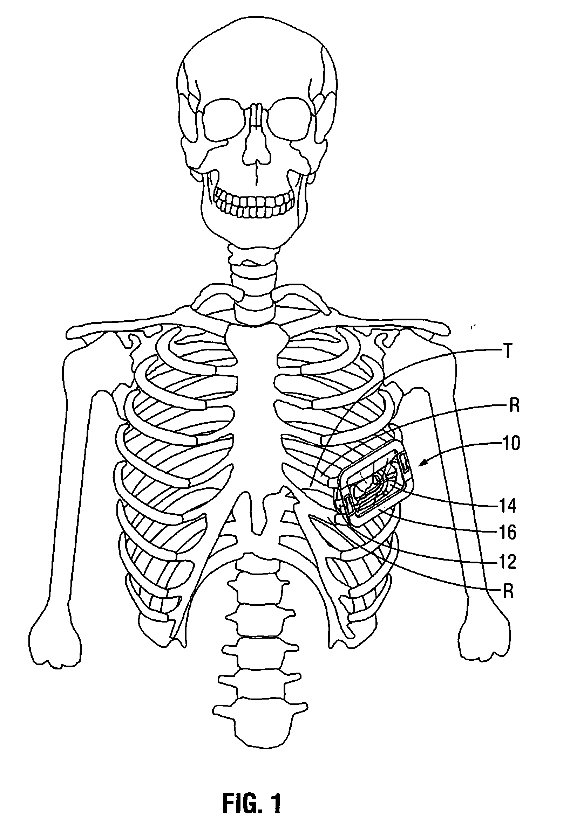

Referring now to FIGS. 1-15, and initially to FIG. 1, the presently disclosed surgical access port is shown generally identified by the reference numeral 10. In the embodiment of FIGS. 1-15, the surgical access port 10 is depicted as a thoracic port 10 that is configured and dimensioned for insertion into the intercostal space located between the adjacent ribs "R" (FIG. 1) of a patient in order to allow for the insertion and manipulation of one or more surgical instruments within the thoracic cavity. However, it is also envisioned that surgical access port 10 may be configured and dimensioned to provide access to a variety of other internal body cavities and/or tissues. Further, surgical access port 10 may be formed from any suitable biocompatible material of strength suitable for the purpose described herein, including, but not being limited to, polymeric materials.

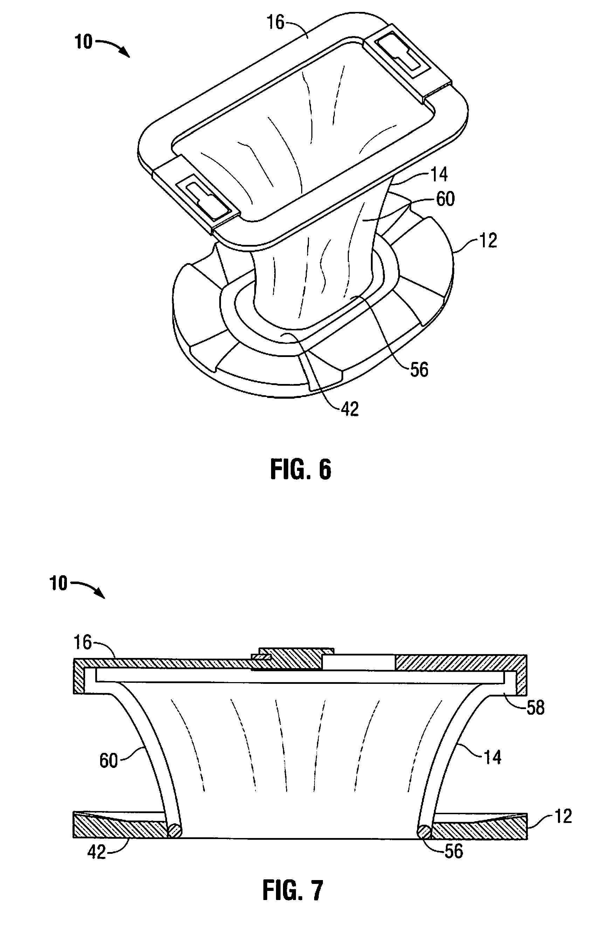

Access port 10 is configured and dimensioned to extend into a body cavity, e.g., the thoracic cavity "T" (FIG. 1) through the intercostal space, and generally includes an inner (distal) ring or body 12 having a flexible member, e.g. membrane 14, coupled to and extending proximally from body 12. An outer (proximal) ring 16 is affixed to a proximal portion of flexible membrane 14 (see FIGS. 6 and 7). The various inner bodies disclosed herein, e.g. body 12, are flexible to enable folding or deformation to a reduced profile for insertion through the incision into the body cavity.

The membrane 14 may be formed from any suitable bio-compatible material having a sufficient elasticity to retract a wide range of tissue depths and to accommodate moving instruments, a sufficient toughness to resist puncturing from sharp instruments, and sufficient strength to be able to retract and oppose thoracic tissue. It can also be made of a transparent material to permit visualization through access port 10 and into the surgical site.

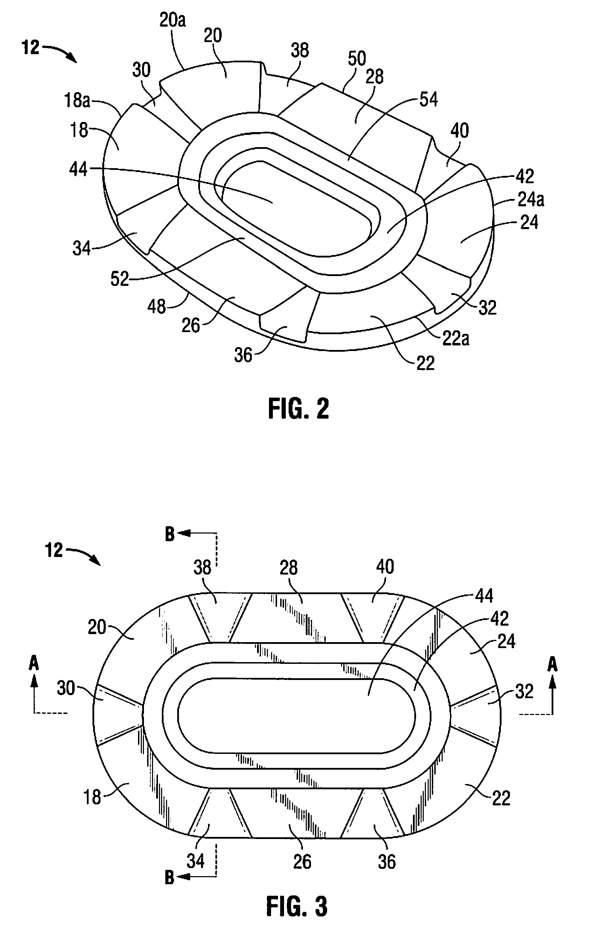

Referring to FIGS. 2-5, body 12 generally includes opposed pairs of curved reinforcement or end panels 18, 20 and 22, 24. Body 12 additionally includes reinforcement or side panels 26 and 28 located between end panels 18, 22 and 20, 24 respectively. Wedge shaped end web sections 30 and 32 connect end panels 18, 20 and 22, 24 respectively. End web sections 30 and 32 are preferably more flexible than end panels 18, 20 and 22, 24 in order to allow body 12 to be folded or flexed during insertion through tissue.

Wedge shaped side web sections 34 and 36 connect side panel 26 to end panels 18 and 22. Wedge shaped side web sections 38 and 40 connect side panel 28 to end panels 20 and 24. Similar to end web sections 30 and 32 described herein above, side web sections 34, 36, 38 and 40 are preferably more flexible than the adjacent panels to facilitate flexing and/or folding of body 12. As shown, an inner ring 42 is provided within body 12 and defines a central passageway or opening 44 for surgical instrumentation.

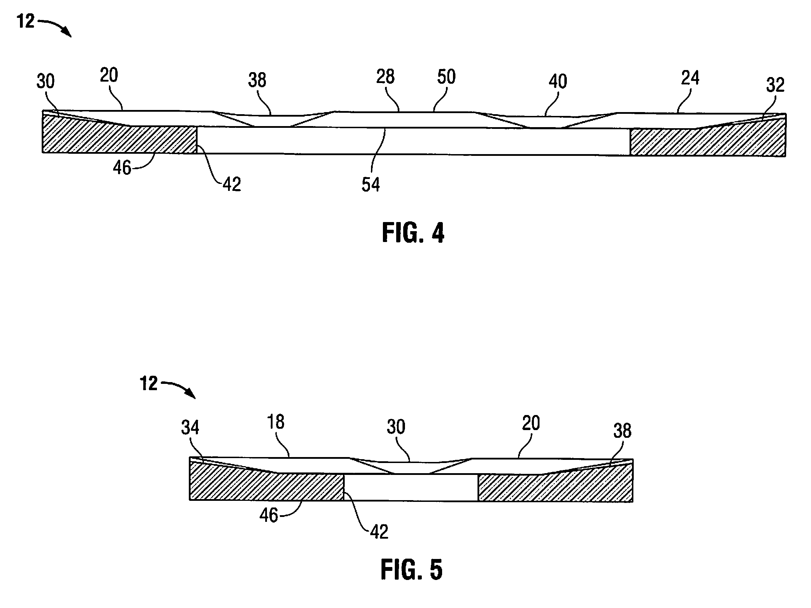

With specific reference now to FIGS. 4 and 5, an underside 46 of inner body 12 provides a surface for affixing flexible membrane 14 (FIG. 1) by various known methods such as, for example, gluing, thermal welding, co-molding, overmolding, etc. In order to allow surgical instrumentation to be more easily manipulated through body 12, the disclosed end and side panels can be thicker at outer edges and tapered to thinner dimensions along the inner edges adjacent inner ring 42. Specifically, outer edges 48 and 50 (see e.g. FIG 2) of side panels 26 and 28 are thicker than inner edges 52 and 54 of side panels 26 and 28, respectively. End panels 18, 20 and 22, 24 also can exhibit this tapering of thickness from a greater dimension along a respective outer edge (18a, 20a, 22a, 24a) to their respective inner edges adjacent inner ring 42.

The thickness variations in the disclosed panels facilitate folding of body 12 during insertion through tissue and provide rigidity to body 12 when body 12 is inserted and positioned against the inside of tissue.

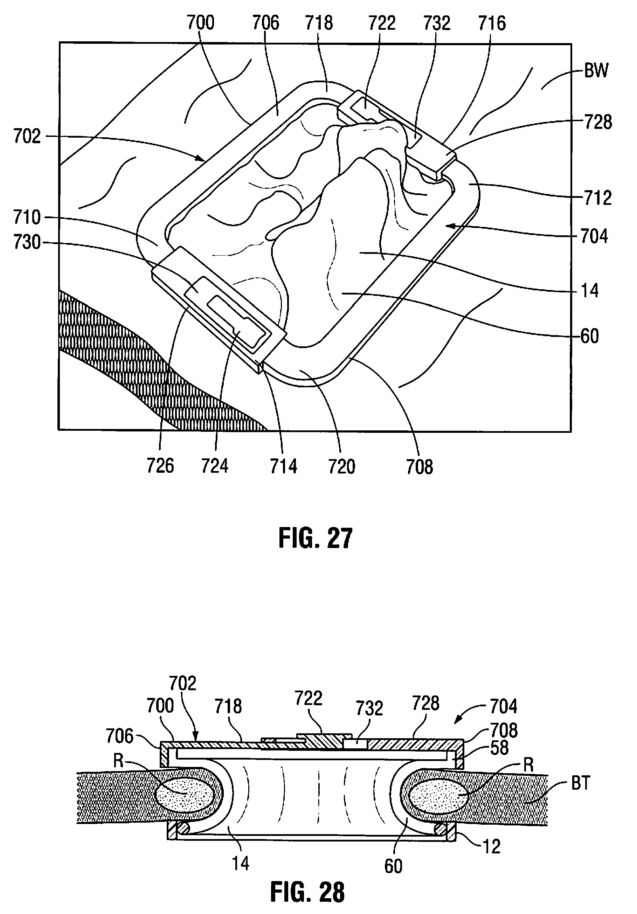

Referring now to FIGS. 6 and 7, as noted hereinabove, surgical access port 10 includes a distal or inner body or frame 12 which is foldable or deformable, a flexible member or membrane 14 and an outer (proximal) ring or frame 16. A lower or distal end 56 of flexible membrane 14 is affixed to inner ring 42 of body 12. An upper or proximal end 58 of flexible membrane 14 is affixed to outer ring 16. A central or middle portion 60 of flexible membrane 14 extends between proximal end 58 and distal end 56 of flexible membrane 14. Outer ring 16 is described in more detail hereinbelow with regard to FIGS. 27-30.

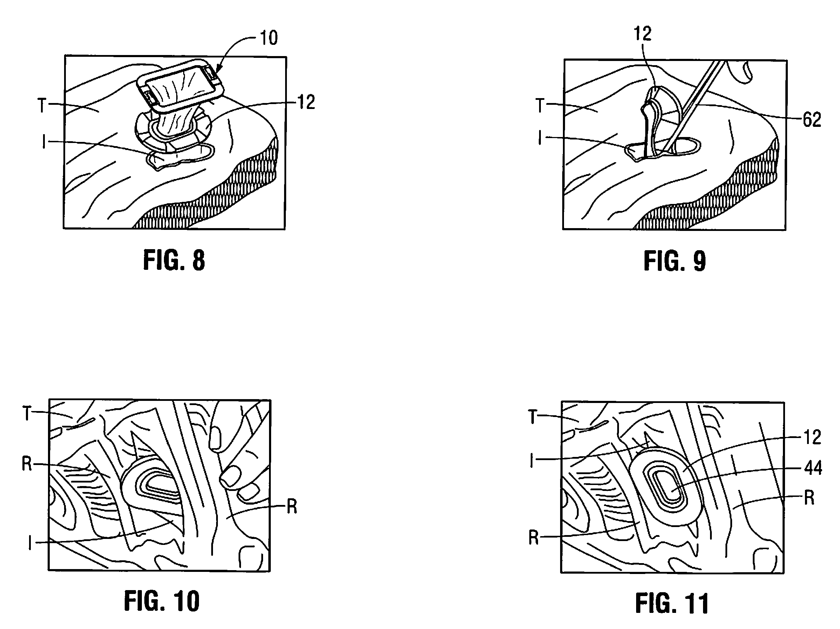

Referring now to FIGS. 8-15, and initially with regard to FIGS. 8-11, the use of surgical access port 10 will now be described. As best shown in FIG. 8, an incision I is formed through the patient's body adjacent the thoracic cavity T. Surgical access port 10 is positioned adjacent incision I and folded by a user's fingers or alternately by a pair of forceps 62 to fold body 12 and insert body 12 through incision I (FIGS. 9 and 10). (Body 12 is illustrated in FIG. 9 in isolation from the remainder of surgical access port 10 for clarity). Once body 12 has passed through incision I, surgical access port 10 is tensioned upwardly to pull body 12 into engagement with adjacent ribs R in thoracic cavity T (FIG. 11). As shown, central opening 44 in body 12 provides an access passageway for the insertion of surgical instruments into thoracic cavity T.

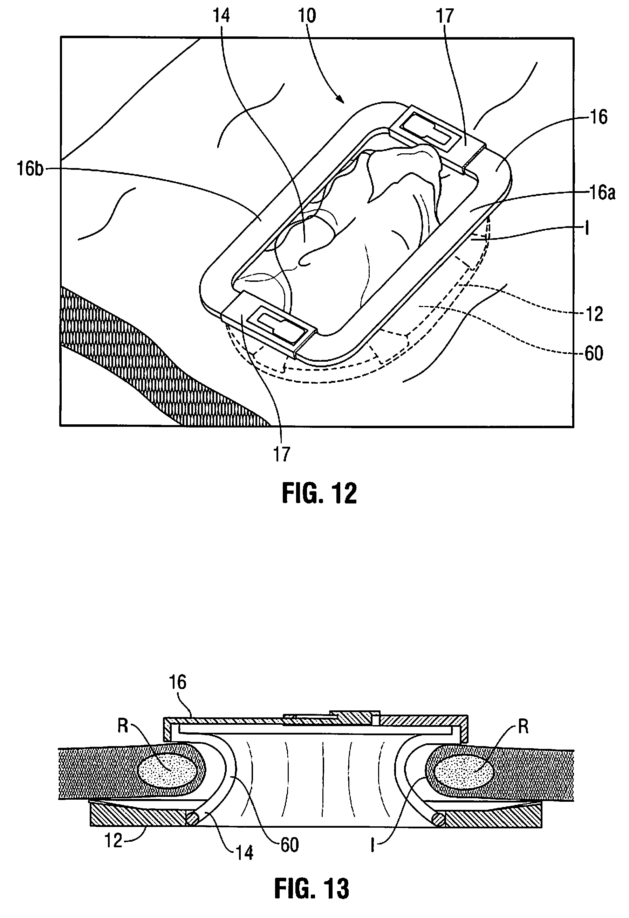

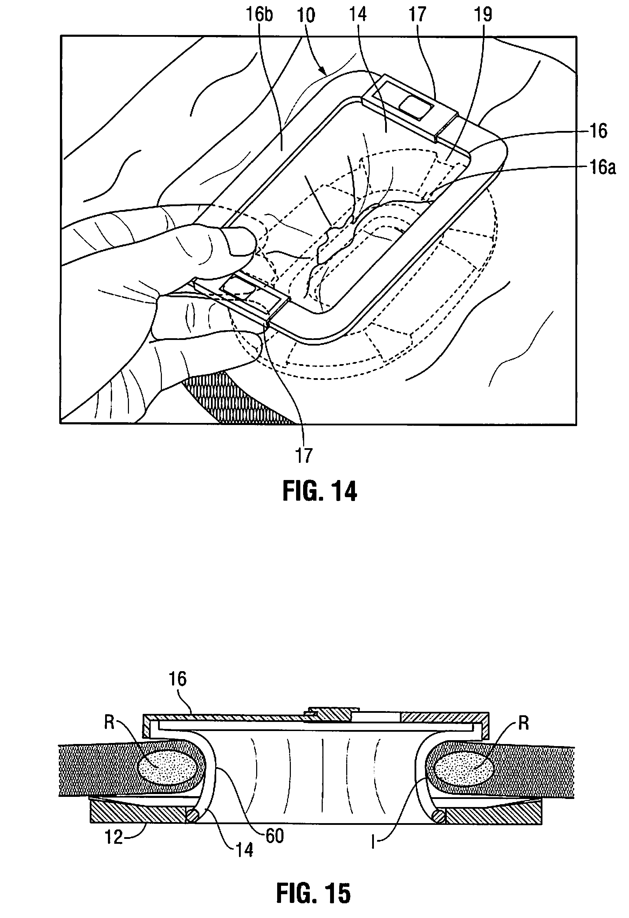

As best shown in FIGS. 12 and 13, surgical access port 10 is properly positioned through incision I in the patient's body with middle portion 60 of flexible membrane 14 engaging tissue at incision I. Flexible membrane 14 is in an untensioned condition. Referring now to FIGS. 14 and 15, in order to enlarge/retract incision I and to create an access passageway through surgical access port 10, outer ring 16 is expanded, e.g. U-shaped frame portions 16a, 16b are moved away from each other, thereby tensioning or expanding flexible membrane 14 to a tensioned (expanded) condition to retract tissue to provide a pathway through opening 44 in body 12. Thereafter, surgical instruments may be inserted through surgical access port 10, specifically through the opening 19 in outer ring 16, the opening in flexible membrane 14 and the opening 44 in body 12 to perform surgical operations. Note frame portions 16a, 16b can be locked in expanded positions by locking structures 17 an opposing sides of outer ring 16. The locking structures 17 can then be released to enable movement of frame portions 16a, 16b to the non-expanded position for removal. An example of locking structures that can be utilized are disclosed in patent application has 13/166,878, filed June 23, 2011 and in patent application 13/297,743, filed November 16, 2011. Once the operations have been completed, body 12 may again be engaged by the user's fingers or by forceps 62 and folded to remove body 12 through incision I. Note Figure 14 illustrates one embodiment of the outer ring 16 which can be utilized, it being understood that other embodiments and other ways to expand and lock the frame to tension the membrane 14 are also contemplated.

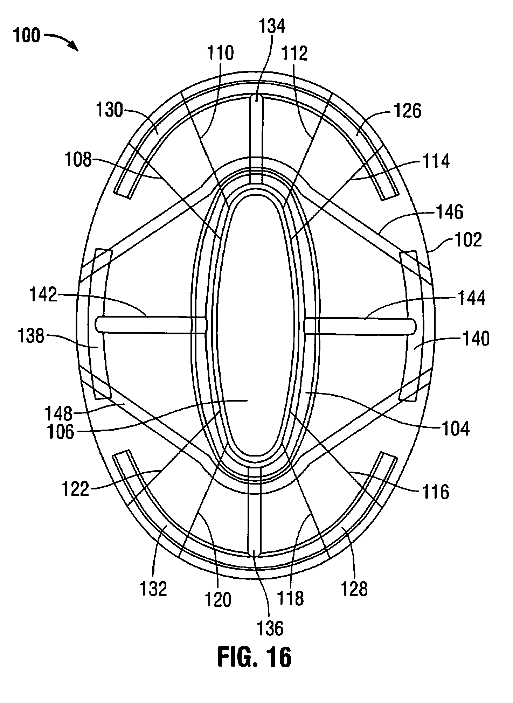

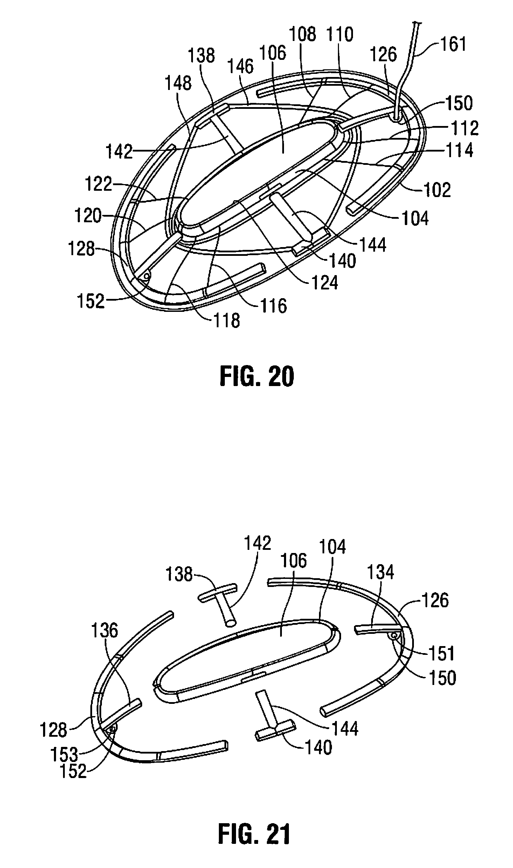

Referring now to FIGS. 16, 20, and 21, there is disclosed an alternative embodiment of an inner (distal) ring or body 100 for use with access port 10 or other access ports. Body 100 of FIG. 16 is generally oval and includes a flexible base portion 102 and an inner ring 104 defining an opening 106. A plurality of weakened areas or fold lines 108, 110, 112, 114 and 116, 118, 120 and 122 are formed adjacent opposed ends of body 100 to facilitate folding body 100 during insertion through an incision in tissue. As best shown in FIG. 20, inner ring 104 includes a raised area or lip 124 to facilitate attachment of flexible membrane 14 described above. Raised area 124 can be seated between the patient's ribs. It should be appreciated that the other inner rings described herein can also have a raised lip similar to lip 124.

Body 100 is provided with opposed pairs of substantially C-shaped reinforcing end ribs 126 and 128. Reinforcing end ribs 126 and 128 generally include curved portions 130 and 132 and are connected to inner ring 104 by respective connecting portions 134 and 136. Ribs 126, 128 preferably correspond to the curvature of the ends of the substantially oval shaped body 12, with connecting portions 134, 136 extending toward the center of body 12 and to raised area 124. Body 100 further includes reinforcing side ribs or portions 138 and 140 which are also connected to inner ring 104 by respective connecting portions 142 and 144. Ribs 138, 140 are on the elongated side of the oval shaped body 12, with connecting portions 142, 144 extending toward the center of body 12 and toward raised area 124. Ribs 138, 140 can be straight or curved. A pair of transverse biasing members or ribs 146 and 148 can be provided to assist in expanding body 100 once positioned within the body of the patient. Biasing ribs 146, 148 are illustrative substantially V-shaped and facing one another on each side of inner ring 104, and each arm extends from a raised area 124 to ribs 138, 140. The center region of ribs 146, 148 can be curved to conform to the curved ends of inner ring 104.

Reinforcing end ribs 126 and 128 along with reinforcing side ribs 138 and 140, including connecting portions 142 and 144, are preferably formed from a material which is stiffer than that of the surrounding flexible base portion 102. The reinforcing members may be formed integrally with inner ring 104 (FIG. 20) or may be provided as separate components affixed to inner ring 104 by various known methods such as, for example, gluing, welding, molding, overmolding etc. Biasing ribs 146, 148 can also be formed integrally with ring 104 or provided as separate components.

Tabs 150 and 152 with respective openings 151, 153 are provided on connecting portions 134 and 136, respectively, for attachment of ribbons or strings to facilitate removal of body 100 from tissue. That is, such ribbon or string (only one string 161 is shown) can be of sufficient length to extend through the incision and outside the patient's body for grasping by the user.

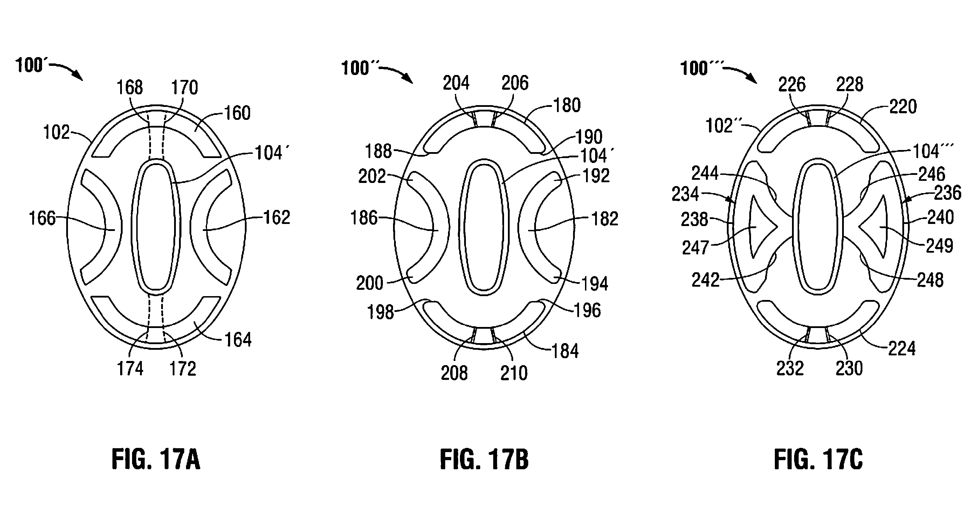

Referring to FIGS. 17A-19B, there are disclosed various alternative embodiments of reinforcing rib layouts or structures for use in body 100 of the surgical access port. The remaining outer ring and membrane can be the same as in FIG. 6. Similar to that disclosed herein above, the following reinforcing rib layouts or structures are formed from materials which are substantially stiffer than surrounding flexible base 102. Further, the reinforcing elements can be formed integrally with inner ring 104 or provided as separate components and at fixed to the inner ring by various methods, including those described above.

Referring to FIG. 17A, four arcuate ribs 160, 162, 164 and 166 are provided around the periphery of body 100'. Ribs 160, 164 adjacent the shorter side of the substantially oval body face toward each other, i.e. toward the center; ribs 162, 166 on the longer side of the body face away from each other, i.e. toward the periphery. Opposed pairs of fold lines 168, 170 and 172, 174 extend across arcuate ribs 160 and 164, respectively. As shown, fold lines 168, 170, 172 and 174 extend to inner ring 104'.

Referring now to FIG. 17B, body 100" has base 102". Body 100" may also be provided with arcuate ribs 180, 182, 184 and 186 around its periphery. In this embodiment, opposite ends 188 and 190, 192, and 194, 196 and 198, and 200 and 202 of respective arcuate ribs 180, 182, 184 and 186 are more rounded than the ends of arcuate ribs 160, 162, 164 and 166 of FIG 17A. Additionally, fold lines 204 and 206 extend across arcuate rib 180 and fold lines 208 and 210 extends across arcuate rib 184. As shown, fold lines 204, 206, 208 and 210 do not extend to inner ring 104" as in the embodiment of FIG. 17A, although in alternate embodiments they can extend to ring 104".

Referring to FIG. 17C, in this embodiment, body 100"' with base 102"' includes an opposed pair of arcuate ribs 220 and 224 which include respective localized fold lines 226, 228, 230 and 232 respectively. As shown, fold lines 226, 228, 230 and 232 do not extend to inner ring 104" similar to that disclosed in the embodiment of FIG. 17B.

In this embodiment, body 100"' is provided with a pair of side or skeletonized, bat wing shaped rib structures 234 and 236. Bat wing ribs structures 234 and 236 include respective arcuate outer ribs 238 and 240 adjacent the periphery of flexible base 104"'. Opposed pairs of arcuate legs 242, 244 and 246, 248 extend from respective outer ribs 238 and 240 to inner ring 104"'. A substantially triangular space 247, 249 is formed in each structure 234, 236, respectively.

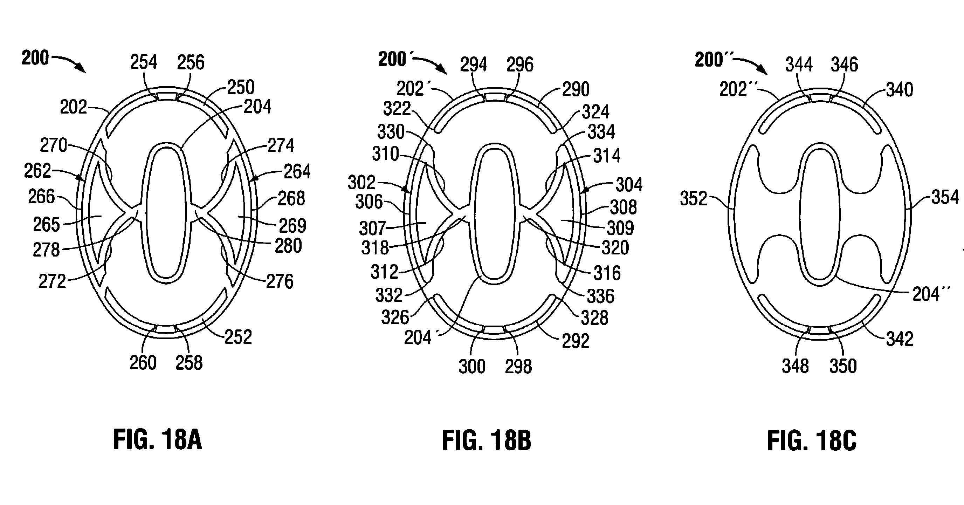

Turning now to FIGS. 18A-18C, and initially to FIG. 18A, in this embodiment, body 200 includes opposed pairs of arcuate ribs 250 and 252, facing each other, which include respective fold lines 254, 256 and 258, 260. Skeletonized bat wing ribs 262 and 264 are provided on flexible base 202 and include respective arcuate outer ribs 266 and 268. Arcuate legs 270, 272 and 274, 276 connect outer ribs 266 and 268 to inner ring 204. Specifically, bulges 278 and 280 extend from inner ring 204 and connect to inner ring 104 and to the arcuate legs of the bat wing ribs 262 and 264. The wing ribs 262,264 in this embodiment are thinner than the wing ribs 234, 236 of FIG. 17C. A substantially triangular shaped space 265, 269 is formed in each of the bat wing ribs 262, 269, respectively.

With reference now to FIG. 18B, in this alternate embodiment, body 200' includes opposed arcuate ribs 290 and 292, facing each other, having respective fold lines 294, 296 and 298, 300. Skeletonized bat wing ribs 302 and 304 are provided on flexible base 202'. Bat wing ribs 302 and 304 include respective outer ribs 306 and 308. Bat wing rib 302 includes arcuate legs 310 and 312 and bat wing rib 304 includes arcuate legs 314 and 316. Bulges 318 and 320 are provided on inner ring 204' and are connected to the arcuate legs of the respective bat wing ribs 302 and 304. Opposed ends 322, 324 and 326, 328 of arcuate ribs 290 and 292, respectively, are rounder than those of arcuate ribs 250 and 252 of FIG. 18A. Likewise, opposed ends 330, 332 and 334, 336 of outer ribs 306 and 308, respectively, are rounder than those of outer ribs 266 and 268 disclosed in FIG. 18A. A substantially triangular shaped space 307, 309 are formed in bat wing ribs 302, 304, respectively.

Turning now to FIG. 18C, body 200" with base 202" includes opposed pairs of arcuate ribs 340 and 342, facing each other, which include respective fold lines 344, 346 and 348, 350. In this embodiment, body 200" additionally includes opposed pairs of monolithic reinforcing structures or ribs 352 and 354 which are formed integrally with, and extend from, inner ring 204". Reinforcing ribs 352 and 354 form bat wing structures and provide additional side stability to body 100 as body is folded along fold lines 344, 346, 348 and 350. Note in this embodiment, the bat wing structures do not have spaces as in the embodiments of FIGS. 18A and 18B.

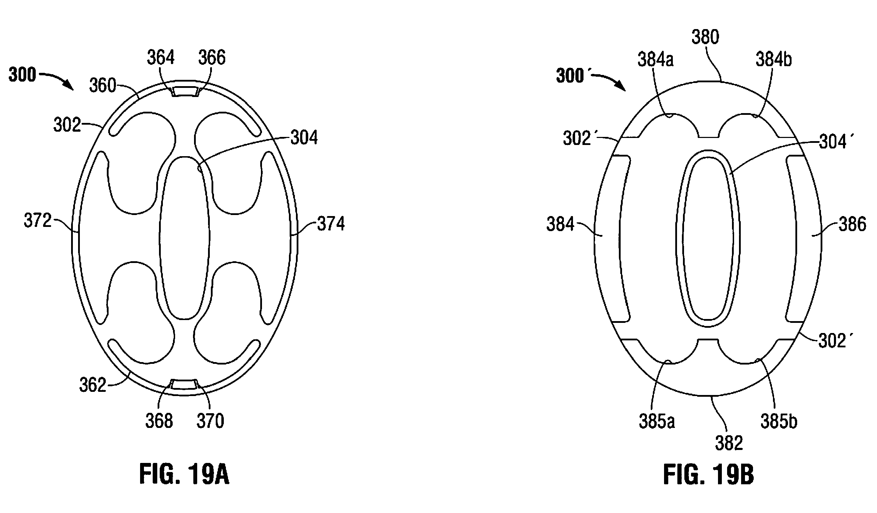

With reference now to FIG. 19A, body 300 with base 302 is provided with opposed pairs of anchor shaped, monolithic reinforcing ribs 360 and 362 extending from inner ring 304 and facing toward each other. Reinforcing ribs 360 and 362 include respective fold lines 364, 366 and 368, 370. Similar to the embodiment disclosed in FIG. 18C above, body 300 additionally includes opposed pairs of monolithic bat wing ribs 372 and 374. As shown, reinforcing ribs 360 and 362 and bat wing ribs 372 and 374 are formed integrally with inner ring 304.

Referring now to FIG. 19B, in this embodiment, body 300' is provided with opposed pairs of truncated, anchor shaped reinforcing ribs 380 and 382 which are not connected to inner ring 304' and face each other. Each rib 380, 382 forms two concave regions, 384a, 384b and 385a, 385b, respectively. Opposed pairs of side ribs 384 and 386, facing each other, are slightly curved and provided on flexible base 302' adjacent the periphery of body 300' and are also not attached to inner ring 304'. Ribs 384, 386 curve to accommodate the curve of body 300'.

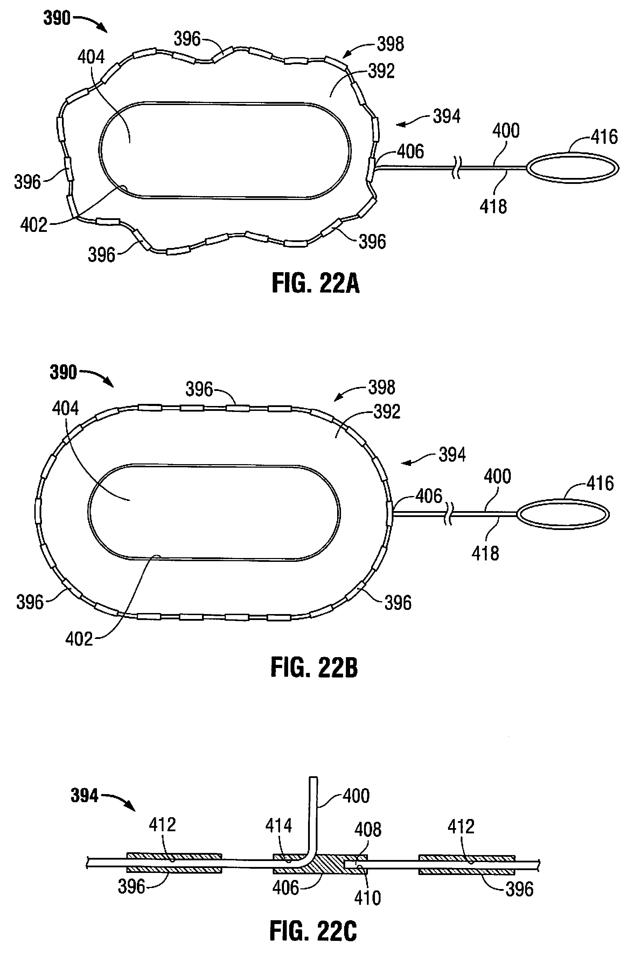

Referring now to FIGS. 22A-C, there is disclosed a collapsible body 390 for use with surgical access port 10 described above. Collapsible body 390 generally includes a flexible base 392 having a tensioning mechanism 394. Tensioning mechanism 394 includes a plurality of ferrules 396 positioned about a periphery 398 of flexible base 392. Tensioning mechanism 394 is provided to alter the shape of body 390 from a collapsed condition (FIG. 22A) to a tensioned or expanded condition (FIG. 22B). Tensioning mechanism 394 additionally includes a tensioning string 400 which freely passes through ferrules 396. Similar to embodiments disclosed above, body 390 also includes an inner ring 402 defining an opening 404 for passage of a surgical instrument. As can be appreciated, smaller gaps between the ferrules 396 are also contemplated so that rather than spread as shown in FIGS. 22A-22C, the ferrules 396 are sufficiently close so that there is only clearance for movement.

Tensioning mechanism 394 additionally includes a base or anchoring ferrule 406. As best shown in FIG. 22C, anchoring ferrule 406 secures a free end 408 of tensioning string 400 within a bore 410. As noted hereinabove, tensioning string 400 freely passes through throughbores 412 formed in ferrules 396. In order to tension and release flexible base 392, anchoring ferrule 406 includes an angled through bore 414 for free passage of tensioning string 400.

In use, body 390 is initially in the collapsed condition (FIG. 22A) and is inserted through an incision formed in a body wall of a patient in a manner described hereinabove. Thereafter, tensioning string 400 is pulled or tensioned thereby drawing tensioning string through angled through bore 414 in anchoring ferrule 406 and throughbores 412 formed through ferrules 396. A loop 416 may be provided at a proximal end 418 of tensioning string 400 to facilitate pulling string 400. As tensioning string 400 is pulled, body 390 moves from the initial, collapsed condition (FIG. 22A) to a final expanded or tensioned condition (FIG. 22B) to engage the underside of a body cavity of the patient and adjacent ribs. After the surgical procedure has been performed through opening 404 in inner ring 402, tension on tensioning string 400 may be released thereby allowing body 390 to move or return to the collapsed condition (FIG. 22A). At this point, body 390 may be removed through the incision formed in the body wall of the patient.

In this manner, collapsible body 390 provides a convenient and simple method of expanding and collapsing a body of surgical access port 10.

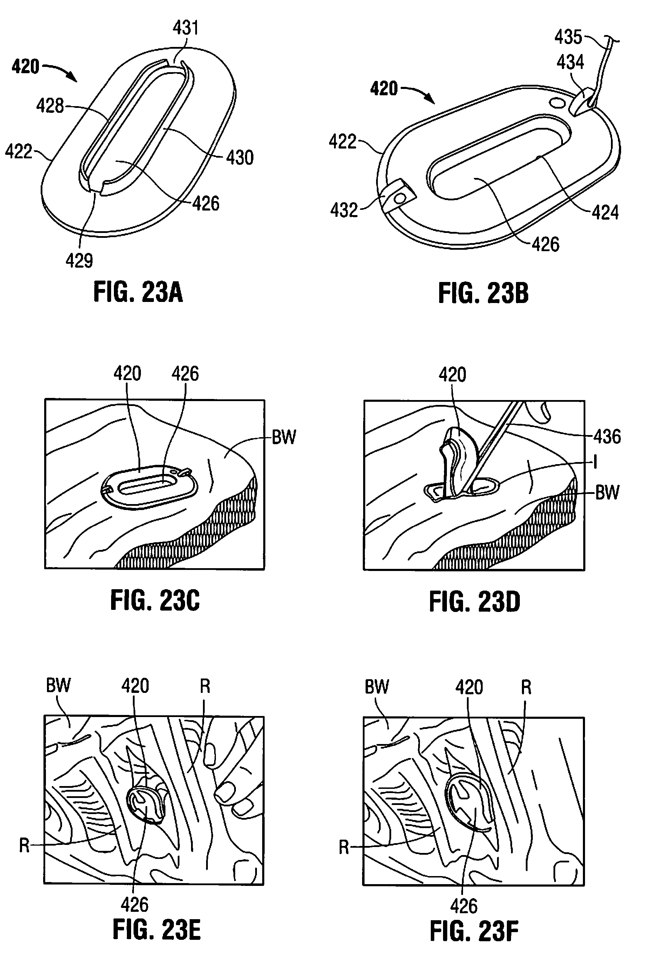

Turning now to FIGS. 23A- 23F, and initially with regard to FIGS. 23A and 23B, there is disclosed an alternative body 420 for use with surgical access port 10 described herein above. Body 420 is formed of a flexible material similar to that disclosed above with respect to bodies 12 and 100 to facilitate folding or deformation for insertion. Body 420 generally includes a flexible base 422 having a central inner ring 424 defining a central opening 426 for passage of surgical instruments. As best shown in FIG. 23A, a pair of side walls 428 and 430 surround opening 426 forming a lip and provide an attachment point for flexible membrane 14 and can also seat between the ribs. Gaps 429 and 431 are provided between side walls 428 and 430 to allow body 420 to be folded longitudinally as it is inserted into an incision in a patient's body.

With reference to FIG. 23B, a pair of tabs 432, 434 may be provided on flexible base 422 for receipt of one or more ribbons or strings (not shown) to facilitate removal of body 420 from the patient after a surgical procedure has been completed. An exemplary ribbon 435 is illustrated. Alternatively, the ribbon can pass through the opening 426 to facilitate removal.

In FIG. 23C, in use, body 420 is initially positioned adjacent a body wall BW. In use as shown in FIG. 23D, body 420 is inserted through an incision I in body wall BW by the use of a pair of forceps 436 (or alternatively by the user's fingers) and by folding body 420 longitudinally in half into a generally "taco" shape.

As shown in FIG. 23E, inserted through body wall BW in minimally invasive thoracic surgery, body 420 resides within the thoracic cavity. The body 420 is pulled upwardly (FIG. 23F) such that side walls 428, 430 extend proximally into the intercostal space of the patient between adjacent ribs R. When positioned, opening 426 (FIG. 23C) provides a passageway for insertion of surgical instruments. The membrane and outer ring are not shown in these drawings for clarity.

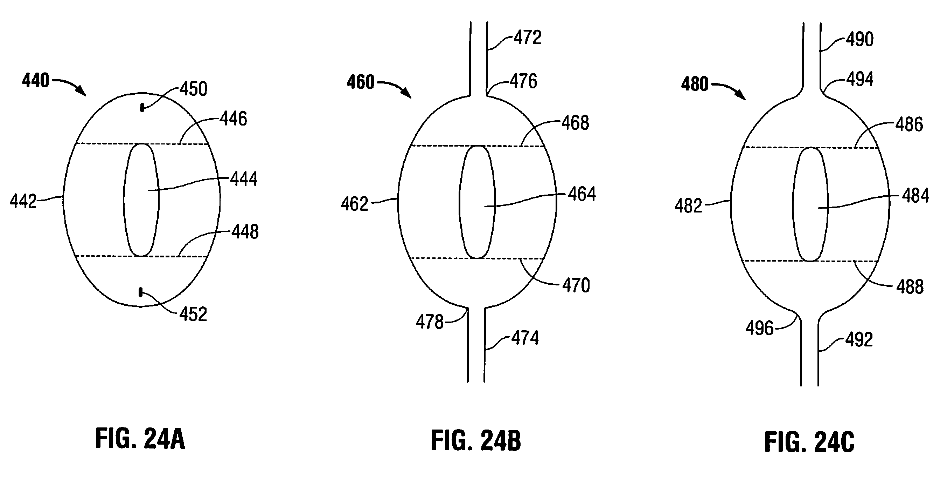

Referring now to FIGS. 24A - 26C, there are disclosed various alternative configurations for a body for use with surgical access port 10. Note these embodiments of FIGS. 24A - 26C can optionally include the reinforcing rib or strut layouts of the various embodiments disclosed herein. Referring to FIG. 24A, a body 440 is provided and includes a substantially oval periphery 442 defining a central substantially oval opening 444. Body 440 additionally includes a pair of transverse fold lines 446 and 448 to facilitate insertion of body 440 into the body of a patient. Body 440 additionally includes a pair of notches 450 and 452 for receipt of ribbons or strings to facilitate maneuvering and removal of body 440.

With regard to FIG. 24B, there is disclosed a body 460 having a substantially oval periphery 462 and a substantially oval central opening 464. Body 460 also includes a pair of transverse fold lines 468 and 470. In order to facilitate removal of body 460, body 460 additionally includes a pair of removal strings or straps 472 and 474. As shown, straps 472 and 474 join periphery 462 of body 460 at relatively sharp transitions 476 and 478.

Turning to FIG. 24C, there is disclosed a body 480 also having a substantially oval periphery 482 and a substantially oval central opening 484. Body 480 additionally includes a pair of transverse fold lines 486 and 488 and removal straps 490 and 492. In this embodiment, removal straps 490 and 492 are connected to periphery 482 at relatively smooth transitions 494 and 496.

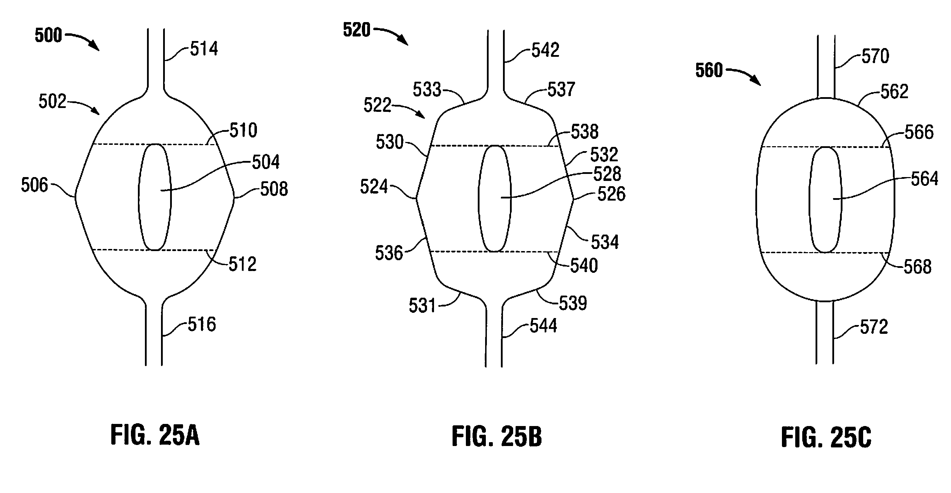

Referring now to FIG. 25A, there is disclosed an alternative body 500 having a semi-oval periphery 502 defining a substantially oval central opening 504. Body 500 includes high points 504 and 506. Body 500 additionally includes transverse fold lines 510 and 512. End straps 514 and 516 extend from the periphery and are provided to facilitate removal of body 500 from the body of a patient. A relatively sharp transition of straps 514, 516 is shown; however, a smoother transition could also be provided.

A further alternative body 520 is shown in FIG. 25B and also has a semi-oval periphery 522 including high points 524 and 526 and a substantially oval central opening 528. Semi-oval periphery 522 is less rounded than body 500 as it includes substantially flat or linear sides 530, 532, 534 and 536. Ends 533, 537 and 531, 539 are less radiused than corresponding ends of body 500 of FIG. 25A. Body 520 additionally includes transverse fold lines 538 and 540 and end straps 542 and 544 which can have a sharp or smooth transition from tip periphery.

Referring to FIG. 25C, there is disclosed a further alternate body 560 having a substantially oval (race track) shape or elongated round periphery 562 and a substantially oval central opening 564. Transverse fold lines 566 and 568 extend across body 560 adjacent central opening 564. Removal straps 570 and 572 are provided and are affixed to body 560 rather than being formed integrally with the body. Alternatively, they can be integral. It should also be appreciated that the straps of the other embodiments disclosed herein can be affixed or integral.

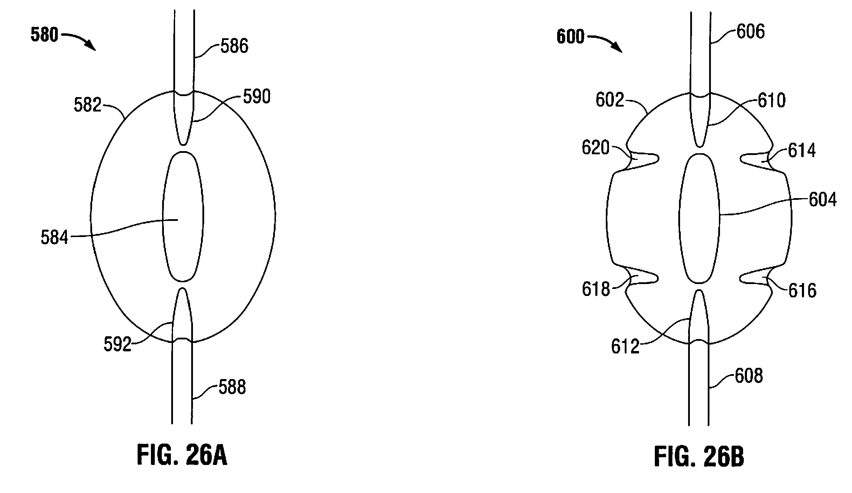

Turning now to FIG. 26A, there is disclosed still a further alternative embodiment of a body 580 for surgical access port 10. Body 580 has a substantially oval periphery 582 and a substantially oval central opening 584. End straps 586 and 588 extend from periphery 582. In this embodiment, end straps 586 and 588 extend from scalloped portions 590 and 592 formed in body 580. Scalloped portions 590 and 592 facilitate folding body 580 longitudinally during insertion and removal from the body of a patient.

Referring now to FIG. 26B, there is disclosed another alternative embodiment of a body 600 for use with surgical access port 100. Body 600 generally includes a substantially oval periphery 602 and a substantially oval central opening 604. End straps 606 and 608 extend from substantially oval periphery 602 and engage substantially oval periphery 602 at a pair of scalloped portions 610 and 612. In order to further facilitate folding of body 600, body 600 additionally includes further scalloped portions 614, 616, 618 and 620 on opposing sides of body 600. The scalloped portions can have uniform depths or varying depths.

Referring now to FIGS. 27-30, an alternative embodiment of the outer ring of the surgical access port of FIG. 6 is disclosed. The outer ring in this embodiment is an offset spacer ring, designated by reference numeral 700. The offset spacer ring 700 can be used with the inner ring of any of the embodiments disclosed herein, as well as with other inner rings. In the illustrated embodiment, the inner ring 12 and membrane 14 are shown. Offset spacer ring 700 is provided to engage upper or proximal end 58 of flexible membrane 14 in order to increase tension on flexible membrane 14 of access port 10 and allow membrane 14 to engage body tissue "BT" (FIGS. 27 and 28). This will minimize wrinkles in flexible membrane 14 and open or expand middle portion 60 of flexible membrane 14 for passage of surgical instruments. The expansion of middle portion 60 functions to also assist in retracting tissue perpendicular to the incision formed through the body tissue "BT". The offset spacer ring 700 raises the height of the external ring off the skin to increase membrane tension.

Offset spacer ring 700 generally includes first and second U-shaped body portions or halves 702 and 704. First and second halves 702 and 704 include respective first and second sections 706 and 708. With reference to FIGS. 27 and 28, first arms 710 and 712 extend from first and second sections 706 and 708, respectively, and terminate in first and second open ends 714 and 716. That is, first arms 710 and 712 include frames 726 and 728 attached thereto which form the open ends. Alternatively, the arms 710 and 712 can be formed with open ends. Second arms 718 and 720 extend from first and second sections 706 and 708 and terminate in first and second insertion tips 722 and 724. Insertion tips 722 and 724 are frictionally received within first and second open ends 714 and 716 of frames 726, 728 of arms 710, 712, in order to adjust the size of offset spacer ring 700 as well as to adjust the tension on flexible membrane 14. If the arms 710, 712 themselves have open ends, then the insertion tips 722 and 724 of arms 718 and 720 would be inserted into these open ends. Interlocking features such as discs or other structures such as these disclosed in application serial nos. 13/166,878, filed June 23, 2011 (publication no. US 2012-0041269 A1) and 13/297,743, filed November 16, 2011 (publication no. US 2012-0143008 A1) can also be utilized for engagement. Optionally frames 726 and 728 can have windows 730 and 732 to allow visualization of insertion tips 722 and 724 as they are inserted into open ends 714 and 716 of first arms 710 and 712.

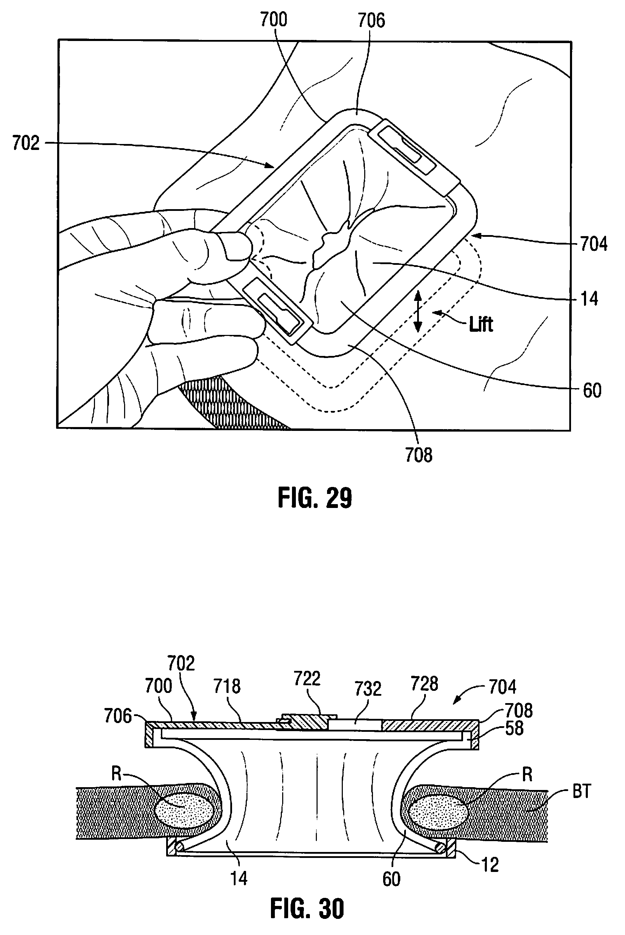

First and second sections 706 and 708 are affixed to proximal end 58 of flexible membrane 14 (FIG. 28). As best shown in FIG. 27, flexible membrane 14 is not in a fully tensioned condition and may include wrinkling or bunching of middle portion 60 of flexible membrane 14. Once body 12 has been positioned through the patient's body, offset spacer ring 700 may be lifted away from body tissue BT (FIG. 29) to thereby tension middle portion 60 of flexible membrane 14 and minimize any wrinkling or bunching of the membrane material. The body halves 702 and 704 are then spread to tension the membrane material and the structure maintains the body halves 702, 704 in the select spaced position.

As best shown in FIG. 30, tensioning of middle portion 60 of flexible membrane 14 both opens middle portion 60 for the passage of surgical instruments, and causes middle portion 60 to engage and retract body tissue BT between adjacent ribs R. Additional structure can be provided to help maintain the spacer ring 700 in the lifted (raised) position.

In this manner, offset spacer ring 700 provides a useful device for establishing and maintaining tension on flexible membrane 14 and retracting tissue adjacent an incision.

## Claims

1. A surgical access assembly (10) for positioning within an opening in tissue, the surgical access assembly comprising:
a flexible body portion (12) positionable within a patient and having an opening (44) and at least one reinforcing member (18, 20, 22, 24, 26, 28), the body portion deformable to a first position for insertion; and
a flexible member (14) coupled to the flexible body portion and extending proximally therefrom;
wherein the at least one reinforcing member includes first and second reinforcing panels (18, 20, 22, 24, 26, 28) separated by a web portion (30, 32, 34, 36, 38, 40), the web portion being more flexible than the first and second reinforcing panels; and
**characterized in that** the at least one reinforcing member includes at least one reinforcing panel tapering from a greater thickness adjacent a periphery of the flexible body portion to a lesser thickness adjacent the opening.

2. The surgical access assembly as recited in claim 1, wherein the at least one reinforcing member includes opposed pairs of reinforcing panels (18, 20, 22, 24) at opposite ends of the flexible body portion.

3. The surgical access assembly as recited in claim 2, wherein the opposed pairs of reinforcing panels (18, 20, 22, 24) have curved configurations.

4. The surgical access assembly as recited in claim 1, wherein the at least one reinforcing member includes opposed pairs of side reinforcing panels (26, 28).

5. The surgical access assembly as recited in claim 1, wherein the at least one reinforcing member is an end rib (126, 128) or a side rib (138, 140).

6. The surgical access assembly as recited in claim 5, wherein the rib is curved.

7. The surgical access assembly as recited in claim 1, wherein the at least one reinforcing member is an opposed pair of end ribs (126, 128).

8. The surgical access assembly as recited in claims 5 or 6, wherein the flexible body portion includes an inner ring (104) defining the opening, and the side rib is connected to the inner ring.

9. The surgical access assembly as recited in claim 8, wherein the side rib has a bat winged shape.

10. The surgical access assembly as recited in claim 9, wherein the bat winged shape rib is skeletonized or the bat wing shaped rib is monolithic.

11. The surgical access assembly as recited in any preceding claim, further comprising an outer ring (16) attached to a proximal portion of the flexible member.

## Patentansprüche

1. Chirurgische Zugangsanordnung (10) für die Positionierung in einer Öffnung in Gewebe, wobei die chirurgische Zugangsanordnung umfasst:
einen flexiblen Körperabschnitt (12), der innerhalb eines Patienten positionierbar ist und eine Öffnung (44) und mindestens ein Verstärkungselement (18, 20, 22, 24, 26, 28) aufweist, wobei der Körperabschnitt in eine erste Position zum Einführen verformbar ist; und
ein flexibles Element (14), das mit dem flexiblen Körperabschnitt verbunden ist und sich proximal davon erstreckt;
wobei das mindestens eine Verstärkungselement erste und zweite Verstärkungsplatten (18, 20, 22, 24, 26, 28) aufweist, die durch einen Stegabschnitt (30, 32, 34, 36, 38, 40) getrennt sind, wobei der Stegabschnitt flexibler ist als die ersten und zweiten Verstärkungsplatten; und
**dadurch gekennzeichnet ist, dass** das mindestens eine Verstärkungselement mindestens eine Verstärkungsplatte aufweist, die sich von einer größeren Dicke angrenzend an einen äußeren Rand des flexiblen Körperabschnitts zu einer geringeren Dicke angrenzend an die Öffnung verjüngt.

2. Chirurgische Zugangsanordnung nach Anspruch 1, wobei das mindestens eine Verstärkungselement gegenüberliegende Paare von Verstärkungsplatten (18, 20, 22, 24) an gegenüberliegenden Enden des flexiblen Körperabschnitts aufweist.

3. Chirurgische Zugangsanordnung nach Anspruch 2, wobei die gegenüberliegenden Paare der Versteifungsplatten (18, 20, 22, 24) gebogene Konfigurationen aufweisen.

4. Chirurgische Zugangsanordnung nach Anspruch 1, wobei das mindestens eine Verstärkungselement gegenüberliegende Paare von Seitenverstärkungsplatten (26, 28) aufweist.

5. Chirurgische Zugangsanordnung nach Anspruch 1, wobei das mindestens eine Verstärkungselement ein Endrippe (126, 128) oder eine Seitenrippe (138, 140) ist.

6. Chirurgische Zugangsanordnung nach Anspruch 5, wobei die Rippe gekrümmt ist.

7. Chirurgische Zugangsanordnung nach Anspruch 1, wobei das mindestens eine Verstärkungselement ein gegenüberliegendes Paar von Endrippen (126, 128) ist.

8. Chirurgische Zugangsanordnung nach Anspruch 5 oder 6, wobei der flexible Körperabschnitt einen Innenring (104) aufweist, der die Öffnung definiert, und die Seitenrippe mit dem Innenring verbunden ist.

9. Chirurgische Zugangsanordnung nach Anspruch 8, wobei die Seitenrippe eine Fledermausflügelform aufweist.

10. Chirurgische Zugangsanordnung nach Anspruch 9, wobei die fledermausflügelförmige Rippe skelettiert oder die fledermausflügelförmige Rippe monolithisch ist.

11. Chirurgische Zugangsanordnung nach einem der vorhergehenden Ansprüche, ferner umfassend einen Außenring (16), der an einem proximalen Abschnitt des flexiblen Elements angebracht ist.

## Revendications

1. Ensemble d'accès chirurgical (10) à positionner à l'intérieur d'une ouverture dans un tissu, l'ensemble d'accès chirurgical comprenant :
une partie formant corps souple (12) pouvant être positionnée à l'intérieur d'un patient et ayant une ouverture (44) et au moins un élément de renforcement (18, 20, 22, 24, 26, 28), la partie formant corps pouvant être déformée jusqu'à une première position pour insertion ; et
un élément souple (14) couplé à la partie formant corps souple et s'étendant de façon proximale à partir de celle-ci ;
dans lequel l'au moins un élément de renforcement inclut des premier et second panneaux de renforcement (18, 20, 22, 24, 26, 28) séparés par une partie formant âme (30, 32, 34, 36, 38, 40), la partie formant âme étant plus souple que les premier et second panneaux de renforcement ; et
**caractérisé en ce que**
l'au moins un élément de renforcement inclut au moins un panneau de renforcement allant en s'amincissant depuis une épaisseur plus grande adjacente à une périphérie de la partie formant corps souple jusqu'à une épaisseur moindre adjacente à l'ouverture.

2. Ensemble d'accès chirurgical selon la revendication 1, dans lequel l'au moins un élément de renforcement inclut des couples opposés de panneaux de renforcement (18, 20, 22, 24) au niveau d'extrémités opposées de la partie formant corps souple.

3. Ensemble d'accès chirurgical selon la revendication 2, dans lequel les couples opposés de panneaux de renforcements (18, 20, 22, 24) ont des configurations curvilignes.

4. Ensemble d'accès chirurgical selon la revendication 1, dans lequel l'au moins un élément de renforcement inclut des couples opposés de panneaux de renforcement latéraux (26, 28).

5. Ensemble d'accès chirurgical selon la revendication 1, dans lequel l'au moins un élément de renforcement est une nervure d'extrémité (126, 128) ou une nervure latérale (138, 140).

6. Ensemble d'accès chirurgical selon la revendication 5, dans lequel la nervure est curviligne.

7. Ensemble d'accès chirurgical selon la revendication 1, dans lequel l'au moins un élément de renforcement est un couple opposé de nervures d'extrémité (126, 128).

8. Ensemble d'accès chirurgical selon les revendications 5 ou 6, dans lequel la partie formant corps souple inclut une bague intérieure (104) définissant l'ouverture, et la nervure latérale est reliée à la bague intérieure.

9. Ensemble d'accès chirurgical selon la revendication 8, dans lequel la nervure latérale a une forme d'aile de chauve-souris.

10. Ensemble d'accès chirurgical selon la revendication 9, dans lequel la nervure en forme d'aile de chauve-souris est évidée ou la nervure en forme d'aile de chauve-souris est monolithique.

11. Ensemble d'accès chirurgical selon n'importe quelle revendication précédente, comprenant en outre une bague extérieure (16) attachée à une partie proximale de l'élément souple.
